# EUROPEAN PATENT SPECIFICATION

(11) **EP 2 896 687 B1**
(45) Date of publication and mention of the grant of the patent: **26.06.2019**
(21) Application number: 13837501.9
(22) Date of filing: 12.09.2013
(51) Int. Cl.: C12N 5/071, C12N 5/00

(54) **METHOD FOR PRODUCING 3D CELL CULTURE**
VERFAHREN ZUR HERSTELLUNG EINER 3D-ZELLKULTUR
PROCÉDÉ DE FABRICATION DE CORPS DE CULTURE CELLULAIRE TRIDIMENSIONNELLE

(30) Priority: 14.09.2012 JP 2012203154
(43) Date of publication of application: 22.07.2015
(73) Proprietor: Osaka University, Suita-shi, Osaka 565-0871 (JP); Mitsubishi Paper Mills Limited, Tokyo 130-0026 (JP)
(72) Inventor: AKASHI, Mitsuru, Suita-shi Osaka 565-0871 (JP); MATSUSAKI, Michiya, Suita-shi Osaka 565-0871 (JP); MATSUZAWA, Atsushi, Tokyo 130-0026 (JP)
(74) Representative: Dehns
(86) International application number: PCT/JP2013/074743
(87) International publication number: WO 2014/042225

(56) References cited:
- WO-A1-2004/078955
- WO-A1-2011/033260
- JP-A- 2006 519 023
- JP-A- 2012 029 684
- JP-A- 2012 115 254
- NISHIGUCHI AKIHIRO ET AL: "Rapid construction of three-dimensional multilayered tissues with endothelial tube networks by the cell-accumulation technique.", ADVANCED MATERIALS (DEERFIELD BEACH, FLA.) 16 AUG 2011, vol. 23, no. 31, 16 August 2011 (2011-08-16), pages 3506-3510, XP002753112, ISSN: 1521-4095
- MATSUZAWA ATSUSHI ET AL: "Construction of three-dimensional liver tissue models by cell accumulation technique and maintaining their metabolic functions for long-term culture without medium change.", JOURNAL OF BIOMEDICAL MATERIALS RESEARCH. PART A APR 2015, vol. 103, no. 4, 12 August 2014 (2014-08-12), pages 1554-1564, XP002753113, ISSN: 1552-4965

## Description

### Technical Field

The present disclosure relates to a method for producing a three-dimensional cell culture construct.

### Background Art

In recent years, regenerative medicine has attracted considerable attention as new medical care, and various methods have been proposed to construct a three-dimensional tissue of cells (e.g., Patent Documents 1 and 2). Patent Document 1 discloses a technology including the steps of (a) forming a cell layer; (b) bringing the cell layer into contact with a solution containing a first material and a solution containing a second material alternately; (c) repeating the step (a) and the step (b); and (d) laminating the cell layers via an extracellular matrix. Patent Document 2 discloses a technology including coating the entire surface of cultured cells with an adhesive film, and culturing the coated cells with the adjacent coated cells being bound together.

### Prior Art Documents

### Patent Documents

Patent Document 1: JP 2007-228921 A
Patent Document 2: JP 2012-115254 A

Akihiro et al., (2011) Advanced Materials 23(31): 3506-3510 discloses the rapid construction of three-dimensional multilayered tissues with endothelial tube netowrks by the cell-accumulation technique. Disclosed is the production of a three-dimensional cell culture construct by culturing coated cells with replacement of the culture medium after 12 hours being preferred as compared to the replacement of the culture medium after 24 hours.

JP 2012115254 A discloses D4 achieving three-dimensional cell culture constructs in a short amount of time, preferably within 24 hours.

### Problem to be Solved by the Invention

In the field of tissue engineering and regenerative medicine, the construction and application of a three-dimensional cell culture construct have been expected. Therefore, the present disclosure provides a method for producing a three-dimensional cell culture construct, which allows a three-dimensional cell culture construct with high activity to be produced.

### Means for Solving Problem

The present disclosure relates to a method for producing a three-dimensional cell culture construct including at least two or more laminated cell layers. Disclosed is a method including the following: seeding coated cells in which a cell surface is coated with a coating film containing an extracellular matrix component; culturing the seeded coated cells in a culture medium; and using at least a portion of the culture medium continuously for 5 days or more.

The present disclosure relates to a method for producing a three-dimensional cell culture construct including at least two or more laminated cell layers. The method includes the following: seeding coated cells in which a cell surface is coated with a coating film containing an extracellular matrix component; and culturing the seeded coated cells in a culture medium. The culture process of the coated cells includes culturing the coated cells in 70 µL or more of the culture medium per 1 × 10⁴ cells.

The present invention provides a method as defined in the appended claims. Thus, the present invention provides a method for producing a three-dimensional cell culture construct comprising at least two or more laminated cell layers, the method comprising:
seeding coated cells in which a cell surface is coated with a coating film containing an extracellular matrix component; and
culturing the seeded coated cells in a culture medium,
wherein the culture process of the coated cells includes culturing the coated cells in 170 µL or more of the culture medium per 1 × 10⁴ cells.

The present disclosure can provide a three-dimensional cell culture construct with high activity.

### Brief Description of Drawings

FIG. 1 is a graph showing the absorbances (560 nm) of a solution in the inside and the outside of an insert before the replacement of a culture medium.
FIG. 2 is a graph showing the gene expression levels of CYP1A1, CYP1A2, ALB, and CYP3A4 after culture for 9 days under various conditions.
FIG. 3 is a graph showing the gene expression levels of CYP1A1 and ALB after culture for 9 days under various conditions.
FIG. 4 is a graph showing changes in the gene expression levels of CYP1A1 and ALB over time.
FIG. 5 shows the microphotographs of three-dimensional cell culture constructs produced in Examples 3 to 5.
FIG. 6 is a graph showing changes in the gene expression levels of ALB and CYP1A1 over time.

Disclosed are findings that coated cells in which a cell surface is coated with a coating film containing an extracellular matrix component are cultured using at least a portion of a culture medium continuously for 5 days or more and/or cultured in 70 µL or more of a culture medium per 1 × 10⁴ cells, thereby producing a three-dimensional cell culture construct with high activity.

The details of the mechanism of formation of a three-dimensional cell culture construct with high activity is unclear, but can be estimated as follows. Cells and a three-dimensional cell culture construct are generally produced by replacing a culture medium every day or two. It is reported that when a three-dimensional cell culture construct is produced by culturing coated cells in which a cell surface is coated with a coating film containing an extracellular matrix component, the number of layers of the three-dimensional cell culture construct obtained is larger in the case where the culture medium is replaced after the culture for 12 hours than in the case where the culture medium is replaced after the culture for 24 hours (e.g., Adv. Mater. 2011, 23, 3506-3510). Liquid factors such as cytokine generated by the coated cells themselves are accumulated in the culture medium, in which the coated cells are being cultured. Replacing the culture medium removes the liquid factors. When the coated cells are cultured by using the culture medium continuously for at least 5 days without replacement, the liquid factors are accumulated in the culture medium. Then, the coated cells are cultured while receiving the liquid factors accumulated in the culture medium, and thus a three-dimensional cell culture construct with higher activity may be provided. Moreover, the volume of the culture medium is 70 µL or more per 1 × 10⁴ cells. This can reduce damage to the three-dimensional cell culture construct due to waste products accumulated in the culture medium. Since the amount of the liquid factors accumulated in the culture medium is increased, the activity of the three-dimensional cell culture construct may be improved. However, the present disclosure is not limited to the above estimation.

In one or more embodiments, the "three-dimensional cell culture construct with high activity" in the present specification means that the cells constituting the three-dimensional cell culture construct have high activity, and/or that the three-dimensional cell culture construct is likely to maintain a three-dimensional form. In one or more embodiments, when the activity of the cells constituting the three-dimensional cell culture construct is high, e.g., the expression level of genes related to metabolism is high. In one or more embodiments, when the three-dimensional form of the three-dimensional cell culture construct is likely to be maintained, e.g., the thickness and/or the number of layers of the three-dimensional cell culture construct obtained is increased.

The "three-dimensional cell culture construct" in the present specification is the assembly of cells and an extracellular matrix, in which the cells are arranged in at least two layers via the extracellular matrix. The "three-dimensional cell culture construct including two or more laminated cell layers" in the present specification indicates that it is not a cell culture construct having a single cell layer. Moreover, the three-dimensional cell culture construct may include a structure of cells in which the cells adhere to each other and grow without being attached to a substrate. The three-dimensional cell culture construct may include one type or two or more types of cells.

The "coated cells" in the present specification include cells and a coating film containing an extracellular matrix component, in which the surface of the cells is coated with the coating film. In one or more non-limiting embodiments, the cells to be coated may be, e.g., cultured cells. The cultured cells are human cells or cells other than human cells. Examples of the cultured cells include primary cultured cells, subcultured cells, and cell line cells. In one or more embodiments, the cells may be adherent cells such as fibroblasts, cancer cells such as hepatoma cells, epithelial cells, vascular endothelial cells, lymphatic endothelial cells, nerve cells, tissue stem cells, embryonic stem cells, and immune cells. The cells may be derived from human cells or cells other than human cells. The cells may be either one type or two or more types. In one or more embodiments, the coated cells can be prepared by a method disclosed in JP 2012-115254 A.

The "extracellular matrix component" in the present specification is a substance that is filled into a space outside of the cells in a living body and has the functions of serving as a framework, providing a scaffold, and/or holding biological factors. The extracellular matrix component may further include a substance that can have the functions of serving as a framework, providing a scaffold, and/or holding biological factors in in vitro cell culture.

In one or more embodiments, the "coating film containing an extracellular matrix component" in the present specification preferably includes a film containing a material A and a film containing a material B that interacts with the material A. In one or more embodiments, the combination of the material A and the material B may be (i) a combination of a protein or polymer having an RGD sequence (also referred to as a "material having an RGD sequence" in the following) and a protein or polymer that interacts with the protein or polymer having an RGD sequence (also referred to as an "interacting material" in the following) or (ii) a combination of a protein or polymer having a positive charge (also referred to as a "material having a positive charge" in the following) and a protein or polymer having a negative charge (also referred to as a "material having a negative charge" in the following).

### [First method for producing three-dimensional cell culture construct]

Disclosed is a method for producing a three-dimensional cell culture construct including at least two or more laminated cell layers. The method includes the following: seeding coated cells in which a cell surface is coated with a coating film containing an extracellular matrix component; culturing the seeded coated cells in a culture medium; and using at least a portion of the culture medium continuously for 5 days or more. This disclosed method is also referred to as a "first production method" in the following. The first production method can provide a three-dimensional cell culture construct with high activity.

The first production method includes culturing the coated cells by using at least a portion of the culture medium continuously for 5 days or more. "Culturing the coated cells by using at least a portion of the culture medium continuously for 5 days or more" in the present disclosure means that the coated cells are cultured while 20% or more, 30% or more, 40% or more, 50% or more, 60% or more, 70% or more, 80% or more, 90% or more, or substantially 100% of the culture medium, in which the coated cells are cultured, continues to be used for at least 5 days. The above disclosed culture process also includes culturing the coated cells without removing 20% or more, 30% or more, 40% or more, 50% or more, 60% or more, 70% or more, 80% or more, 90% or more, or substantially 100% of the culture medium, in which the coated cells are cultured, for at least 5 days. The first production method may include adding a new culture medium during the culture of the coated cells. The period in which at least a portion of the culture medium is continuously used is at least 5 days. The period may be 6 days or more, 7 days or more, 8 days or more, 9 days or more, 10 days or more, 15 days or more, 20 days or more, or 25 days or more. The upper limit of the period of continuous use is not particularly limited and may be appropriately determined, e.g., by the volume of the culture medium, the number of cells to be seeded, and the type of cells. The upper limit may be 90 days or less, 60 days or less, or 40 days or less. In the first production method, in one or more embodiments, the coated cells may be cultured without replacing the culture medium for at least 5 days, or the cycle of replacing the culture medium may be 5 days or more. The first production method may include replacing the culture medium for the first time (first culture medium replacement) after at least 5 days from the start of the culture, or replacing the culture medium for the second time after at least 6 days from the start of the culture. The "culture medium replacement" in the present disclosure means that approximately the total volume of the culture medium, in which the coated cells are cultured, is removed and replaced with a new culture medium.

The first production method may include replacing the culture medium after 16 to 36 hours from the seeding of the coated cells.

The volume of the culture medium during the culture is not particularly limited, but is preferably 70 µL or more, and more preferably 170 µL or more or 1 mL or more per 1 × 10⁴ cells in terms of reducing the influence of waste products included in the culture medium during the culture. The volume of the culture medium may be 100 mL or less or 10 mL or less per 1 × 10⁴ cells.

The first production method includes using at least a portion of the culture medium continuously for 5 days or more, and culturing the coated cells in 70 µL or more of the culture medium per 1 × 10⁴ cells. The first production method includes culturing the coated cells in 70 µL or more of the culture medium per 1 × 10⁴ cells while at least a portion of the culture medium continues to be used.

The culture medium is not particularly limited and may be appropriately determined in accordance with the cells. Examples of the culture medium include Eagle's MEM medium, Dulbecco's Modified Eagle medium (DMEM), Modified Eagle medium (MEM), Minimum Essential medium, RDMI, GlutaMAX medium, and serum-free medium.

The coated cells may be seeded so that at least two layers of the coated cells are formed. The density of the coated cells during seeding may be appropriately determined, e.g., by the thickness of an intended three-dimensional cell culture construct and/or the number of cells to be layered. In one or more embodiments, the density may be 1 × 10² cells/cm³ to 1 × 10⁹ cells/cm³, 1 × 10⁴ cells/cm³ to 1 × 10⁸ cells/cm³, or 1 × 10⁵ cells/cm³ to 1 × 10⁷ cells/cm³.

The incubation temperature may be 4 to 60°C, 20 to 40°C, or 30 to 37°C.

For ease of handling, it is preferable that the coated cells are cultured on a membrane filter. More preferably, the coated cells are cultured using a culture plate that includes a membrane filter. Further preferably, the coated cells are cultured using a culture plate that includes a housing portion and a base portion, in which the base portion serves as a membrane filter. The housing portion is preferably transparent. These culture plates may be commercial products. The commercial products include Transwell (registered trademark), Cell Culture Insert (trade name), etc.

The pore size of the membrane filter is not particularly limited as long as the cultured cells can remain on the membrane filter. The pore size may be 0.1 µm to 2 µm or 0.4 µm to 1.0 µm. The material of the membrane filter may be, e.g., polyethylene terephthalate (PET), polycarbonate, or polytetrafluoroethylene (PTFE).

When the coated cells are cultured by placing an insert in a container such as a well, the ratio of the base area of the container holding the insert to the base area of the insert (base area of container / base area of insert) may be 7 or more, 30 or more, 100 or more, or 160 or more. Moreover, the ratio may be 16000 or less or 1600 or less.

### [Second method for producing three-dimensional cell culture construct]

In one or more aspects, the present invention relates to a method for producing a three-dimensional cell culture construct including at least two or more laminated cell layers. The method includes the following: seeding coated cells in which a cell surface is coated with a coating film containing an extracellular matrix component; and culturing the seeded coated cells in a culture medium. The culture process of the coated cells includes culturing the coated cells in 170 µL or more of the culture medium per 1 × 10⁴ cells. This method of the invention is also referred to as a "second production method" in the following.

In the second production method, the volume of the culture medium during the culture is 170 µL or more or 1 mL or more per 1 × 10⁴ cells in terms of reducing the influence of waste products included in the culture medium during the culture. Moreover, the volume of the culture medium may be 100 mL or less or 10 mL or less per 1 × 10⁴ cells.

In one or more embodiments, the second production method may include replacing the culture medium. The cycle of replacing the culture medium is not particularly limited. In one or more embodiments, the culture medium may be replaced every other day, every three days, every four days, or every five days. In one or more embodiments, the second production method may include culturing the coated cells by using at least a portion of the culture medium continuously for 5 days or more because this can provide a three-dimensional cell culture construct having a large number of layers and/or a large thickness. In one or more embodiments, the period of continuous use may be 6 days or more, 7 days or more, 8 days or more, 9 days or more, 10 days or more, 15 days or more, 20 days or more, or 25 days or more. Moreover, the period of continuous use may be 90 days or less, 60 days or less, or 40 days or less. In the second production method, in one or more embodiments, the cycle of replacing the culture medium may be 5 days or more.

In one or more embodiments, the second production method may include replacing the culture medium for the first time (first culture medium replacement) since the start of the culture and after 16 to 36 hours from the seeding of the coated cells.

The culture conditions such as the type of the culture medium and the incubation temperature in the second production method are the same as those in the first production method.

In the first and second production methods, the thickness of the coating film containing an extracellular matrix component is preferably 1 nm to 1 × 10³ nm or 2 nm to 1 × 10² nm, and more preferably 3 nm to 1 × 10² nm because this can provide a three-dimensional cell culture construct in which the coated cells are more densely layered. The thickness of the coating film containing an extracellular matrix component can be appropriately controlled, e.g., by the number of films constituting the coating film. The coating film containing an extracellular matrix component is not particularly limited. In one or more embodiments, the coating film may be either a single layer or a multi-layer such as 3, 5, 7, 9, 11, 13, 15 layers or more. The thickness of the coating film may be determined by a method as described in the examples.

The first and second production methods may further include preparing coated cells. The coated cells can be prepared by bringing cells into contact with a solution containing a material A and a solution containing a material B alternately. As described above, the combination of the material A and the material B may be a combination of the material having an RGD sequence and the interacting material or a combination of the material having a positive charge and the material having a negative charge.

### (Material having RGD sequence)

The material having an RGD sequence is a protein or polymer having an "Arg-Gly-Asp" (RGD) sequence, which is an amino acid sequence that is associated with cell adhesion activity. The material "having an RGD sequence" in the present specification may be a material that inherently has an RGD sequence or a material to which an RGD sequence is chemically bound. The material having an RGD sequence is preferably biodegradable.

In one or more embodiments, the protein having an RGD sequence may be, e.g., a conventionally known adhesive protein or a water-soluble protein having an RGD sequence. In one or more embodiments, examples of the adhesive protein include fibronectin, vitronectin, laminin, cadherin, and collagen. In one or more embodiments, examples of the water-soluble protein having an RGD sequence include collagen, gelatin, albumin, globulin, proteoglycan, enzymes, and antibodies, to each of which an RGD sequence is bound.

In one or more embodiments, the polymer having an RGD sequence may be, e.g., a naturally occurring polymer or a synthetic polymer. In one or more embodiments, examples of the naturally occurring polymer having an RGD sequence include water-soluble polypeptide, low molecular weight peptide, polyamino acid such as α-polylysine or ε-polylysine, and sugar such as chitin or chitosan. In one or more embodiments, examples of the synthetic polymer having an RGD sequence include straight-chain, graft, comb, dendritic, or star polymers or copolymers having an RGD sequence. In one or more embodiments, examples of the polymers or copolymers include the following: polyurethane, polycarbonate, polyamide, or copolymers thereof; polyester; poly(N-isopropylacrylamide-co-polyacrylic acid); polyamidoamine dendrimer; polyethylene oxide; poly(ε-caprolactam); polyacrylamide; and poly(methyl methacrylate-γ-polyoxyethylene methacrylate).

Among them, the material having an RGD sequence is preferably fibronectin, vitronectin, laminin, cadherin, polylysine, elastin, collagen to which an RGD sequence is bound, gelatin to which an RGD sequence is bound, chitin, or chitosan. The material having an RGD sequence is more preferably fibronectin, vitronectin, laminin, polylysine, collagen to which an RGD sequence is bound, or gelatin to which an RGD sequence is bound.

### (Interacting material)

The interacting material is a protein or polymer that interacts with the material having an RGD sequence. In one or more embodiments, the term "interact" in the present specification means that the material having an RGD sequence and the interacting material approach each other to the extent that bonding, adhesion, adsorption, or electron transfer can occur chemically and/or physically between them, e.g., due to electrostatic interaction, hydrophobic interaction, hydrogen bond, charge transfer interaction, covalent bond formation, specific interaction between proteins, and/or Van der Waals force. The interacting material is preferably biodegradable.

In one or more embodiments, the protein that interacts with the material having an RGD sequence may be, e.g., collagen, gelatin, proteoglycan, integrin, enzymes, or antibodies. In one or more embodiments, the polymer that interacts with the material having an RGD sequence may be, e.g., a naturally occurring polymer or a synthetic polymer. In one or more embodiments, examples of the naturally occurring polymer that interacts with the material having an RGD sequence include water-soluble polypeptide, low molecular weight peptide, polyamino acid, elastin, sugar such as heparin, heparan sulfate, or dextran sulfate, and hyaluronic acid. In one or more embodiments, examples of the polyamino acid include polylysine such as α-polylysine or ε-polylysine, polyglutamic acid, and polyaspartic acid. In one or more embodiments, examples of the synthetic polymer that interacts with the material having an RGD sequence include straight-chain, graft, comb, dendritic, or star polymers or copolymers having an RGD sequence. In one or more embodiments, examples of the polymers or copolymers include the following: polyurethane, polyamide, polycarbonate, or copolymers thereof; polyester; polyacrylic acid; polymethacrylic acid; polyethylene glycol-graft-polyacrylic acid; poly(N-isopropylacrylamide-co-polyacrylic acid); polyamidoamine dendrimer; polyethylene oxide; poly(ε-caprolactam); polyacrylamide; and poly(methyl methacrylate-γ-polyoxyethylene methacrylate).

Among them, the interacting material is preferably gelatin, dextran sulfate, heparin, hyaluronic acid, globulin, albumin, polyglutamic acid, collagen, or elastin. The interacting material is more preferably gelatin, dextran sulfate, heparin, hyaluronic acid, or collagen. The interacting material is further preferably gelatin, dextran sulfate, heparin, or hyaluronic acid.

The combination of the material having an RGD sequence and the interacting material is not particularly limited and may be a combination of different materials that interact with each other. Specifically, one of the materials may be a polymer or protein having an RGD sequence, and the other may be a polymer or protein that reacts with the polymer or protein having an RGD sequence. In one or more embodiments, examples of the combination of the material having an RGD sequence and the interacting material include the following: fibronectin and gelatin; fibronectin and ε-polylysine; fibronectin and hyaluronic acid; fibronectin and dextran sulfate; fibronectin and heparin; fibronectin and collagen; laminin and gelatin; laminin and collagen; polylysine and elastin; vitronectin and collagen; and RGD-bound collagen or RGD-bound gelatin and collagen or gelatin. Among them, the combination is preferably fibronectin and gelatin, fibronectin and ε-polylysine, fibronectin and hyaluronic acid, fibronectin and dextran sulfate, fibronectin and heparin, or laminin and gelatin. The combination is more preferably fibronectin and gelatin. Each of the material having an RGD sequence and the interacting material may be either one type or two or more types as long as they interact with each other.

### (Material having positive charge)

The material having a positive charge is a protein or polymer having a positive charge. In one or more embodiments, the protein having a positive charge is preferably a water-soluble protein. In one or more embodiments, examples of the water-soluble protein include basic collagen, basic gelatin, lysozyme, cytochrome c, peroxidase, and myoglobin. In one or more embodiments, the polymer having a positive charge may be, e.g., a naturally occurring polymer or a synthetic polymer. In one or more embodiments, examples of the naturally occurring polymer include water-soluble polypeptide, low molecular weight peptide, polyamino acid, and sugar such as chitin or chitosan. In one or more embodiments, examples of the polyamino acid include polylysine such as poly(α-lysine) or poly(ε-lysine), polyarginine, and polyhistidine. In one ore more embodiments, examples of the synthetic polymer include straight-chain, graft, comb, dendritic, or star polymers or copolymers. In one or more embodiments, examples of the polymers or copolymers include the following: polyurethane, polyamide, polycarbonate, or copolymers thereof; polyester; polydiallyldimethylammonium chloride (PDDA); polyallylamine hydrochloride; polyethyleneimine; polyvinylamine; and polyamidoamine dendrimer.

### (Material having negative charge)

The material having a negative charge is a protein or polymer having a negative charge. In one or more embodiments, the protein having a negative charge is preferably a water-soluble protein. In one or more embodiments, examples of the water-soluble protein include acid collagen, acid gelatin, albumin, globulin, catalase, β-lactoglobulin, thyroglobulin, α-lactalbumin, and ovalbumin. The polymer having a negative charge may be, e.g., a naturally occurring polymer or a synthetic polymer. In one or more embodiments, examples of the naturally occurring polymer include water-soluble polypeptide, low molecular weight peptide, polyamino acid such as poly(β-lysine), and dextran sulfate. In one or more embodiments, examples of the synthetic polymer include straight-chain, graft, comb, dendritic, or star polymers or copolymers. In one or more embodiments, examples of the polymers or copolymers include the following: polyurethane, polyamide, polycarbonate, or copolymers thereof; polyester; polyacrylic acid; polymethacrylic acid; polystyrene sulfonic acid; polyacrylamide methylpropane sulfonic acid; carboxy-terminated polyethylene glycol; polydiallyldimethylammonium salt; polyallylamine salt; polyethyleneimine; polyvinylamine; and polyamidoamine dendrimer.

In one or more embodiments, examples of the combination of the material having a positive charge and the material having a negative charge include the following: ε-polylysine salt and polysulfonate; ε-polylysine and polysulfonate; chitosan and dextran sulfate; polyallylamine hydrochloride and polystyrene sulfonate; polydiallyldimethylammonium chloride and polystyrene sulfonate; and polydiallyldimethylammonium chloride and polyacrylate. The combination is preferably ε-polylysine salt and polysulfonate or polydiallyldimethylammonium chloride and polyacrylate. In one or more embodiments, the polysulfonate may be, e.g., poly(sodium sulfonate) (PSS). Each of the material having a positive charge and the material having a negative charge may be either one type or two or more types as long as they interact with each other.

Hereinafter, a preferred method for preparing coated cells will be described. First, cells are brought into contact with a solution A containing a material having an RGD sequence, and then the cells are brought into contact with a solution B containing a material that interacts with the material having an RGD sequence, thereby preparing coated cells. However, the present disclosure should not be limited to the following embodiment.

First, cells are brought into contact with the solution A. Consequently, a film containing the material having an RGD sequence is formed on the surface of the cells, and thus the surface of the cells is coated with the film containing the material having an RGD sequence. The contact between the cells and the solution A can be made, e.g., by applying or adding the solution A to the cells, immersing the cells in the solution A, or dropping or spraying the solution A on the cells. In particular, for ease of operation, it is preferable that the cells are brought into contact with the solution A by immersion in the solution A.

The contact conditions may be appropriately determined, e.g., by the contact process, the type of the material having an RGD sequence and/or the type of cells, and the concentration of the solution. The contact time is preferably 30 seconds to 24 hours, 1 minute to 60 minutes, 1 minute to 15 minutes, 1 minute to 10 minutes, or 1 minute to 5 minutes. The ambient temperature and/or the temperature of the solution A during contact is preferably 4 to 60°C, 20 to 40°C, or 30 to 37°C.

The solution A may contain at least the material having an RGD sequence, and preferably contains the material having an RGD sequence and a solvent or a dispersion medium (also simply referred to as a "solvent" in the following). The content of the material having an RGD sequence in the solution A is preferably 0.0001 to 1 mass%, 0.01 to 0.5 mass%, or 0.02 to 0.1 mass%. The solvent may be, e.g., an aqueous solvent such as water, phosphate buffered saline (PBS), or buffer. Examples of the buffer include the following: Tris buffer such as Tris-HCl buffer; phosphate buffer; HEPES buffer; citrate-phosphate buffer; glycylglycine-sodium hydroxide buffer; Britton-Robinson buffer; and GTA buffer. The pH of the solvent is not particularly limited. The pH is preferably 3 to 11, 6 to 8, or 7.2 to 7.4.

The solution A may further contain salt, a cell growth factor, cytokine, chemokine, hormone, biologically active peptide, or a pharmaceutical composition. Examples of the pharmaceutical composition include a therapeutic agent for diseases, a preventive, an inhibitor, an antibacterial agent, and an anti-inflammatory agent. Examples of the salt include sodium chloride, calcium chloride, sodium hydrogencarbonate, sodium acetate, sodium citrate, potassium chloride, dibasic sodium phosphate, magnesium sulfate, and sodium succinate. The salt may be either one type or two or more types. Both the solution A and the solution B may contain the salt, or one of them may contain the salt. The salt concentration in the solution A is not particularly limited. The salt concentration is, e.g., 1 × 10⁻⁶ M to 2 M, preferably 1 × 10⁻⁴ M to 1 M, and more preferably 1 × 10⁻⁴ M to 0.05 M.

Next, the material that has not been used for the formation of the film containing the material having an RGD sequence is separated. The separation may be performed, e.g., by centrifugation or filtration. When the material is separated by centrifugation, e.g., the solution A in which the cells are dispersed is centrifuged, and then the supernatant is removed. The centrifugation conditions may be appropriately determined, e.g., by the type of cells, the concentration of cells, and the composition of the materials contained in the solution A.

In addition to the above separation, a washing operation is preferably performed. The washing operation may be performed, e.g., by centrifugation or filtration. The washing operation may be performed by centrifugation, e.g., a solvent is added to the cells from which the supernatant has been removed, and this solution is centrifuged so that the supernatant is removed. It is preferable that the solvent used for washing is the same as that of the solution A.

Next, the cells coated with the film containing the material having an RGD sequence are brought into contact with the solution B. Consequently, a film containing the interacting material is formed on the surface of the film containing the material having an RGD sequence, and thus the surface of the cells, which has been coated with the film containing the material having an RGD sequence, is further coated with the film containing the interacting material. The contact between the cells and the solution B can be made in the same manner as the contact between the cells and the solution A except that the interacting material is used instead of the material having an RGD sequence.

By repeatedly bringing the cells into contact with the solution A and the solution B alternately, the film containing the material having an RGD sequence and the film containing the interacting material can be alternately laminated to form a coating film containing an extracellular matrix component on the entire surface of the cells. The number of times of the contact between the cells and the solution A or the solution B may be appropriately determined, e.g., by the thickness of the coating film containing an extracellular matrix component to be formed.

### [Three-dimensional cell culture construct]

The present disclosure relates to a three-dimensional cell culture construct that includes cells arranged in at least two layers and an extracellular matrix component, and that is produced by the first or second production method. The three-dimensional cell culture construct can have high activity. The cells and the extracellular matrix component of the three-dimensional cell culture construct are as described above.

### [Method for culturing coated cells]

Disclosed is a method for culturing coated cells that includes the following: seeding coated cells in which a cell surface is coated with a coating film containing an extracellular matrix component; culturing the seeded coated cells in a culture medium; and using at least a portion of the culture medium continuously for 5 days or more. Disclosed is a method for culturing coated cells that includes the following: seeding coated cells in which a cell surface is coated with a coating film containing an extracellular matrix component; and culturing the seeded coated cells in a culture medium. The culture process of the coated cells includes culturing the coated cells in 70 µL or more of the culture medium per 1 × 10⁴ cells. The culture conditions or the like in the culture method of the coated cells are as described above.

### [Method for culturing three-dimensional cell culture construct]

Disclosed is a method for producing a three-dimensional cell culture construct including at least two or more laminated cell layers. The method includes the following: seeding coated cells in which a cell surface is coated with a coating film containing an extracellular matrix component; culturing the seeded coated cells in a culture medium; and using at least a portion of the culture medium continuously for 5 days or more. The present invention provides a method for producing a three-dimensional cell culture construct including at least two or more laminated cell layers, the method comprising the following: seeding coated cells in which a cell surface is coated with a coating film containing an extracellular matrix component; and culturing the seeded coated cells in a culture medium, wherein the culture process of the coated cells includes culturing the coated cells in 170 µL or more of the culture medium per 1 × 10⁴ cells. The culture conditions or the like in the culture method of the three-dimensional cell culture construct are as described above.

Hereinafter, the present disclosure will be described in more detail by way of examples and comparative examples. However, the present disclosure is not limited to the following examples.

### Examples

### [Preparation of coated cells]

HepG2 cells were collected from a petri dish by a trypsin treatment. The collected cells were dispersed at a concentration of 1 × 10⁷ cell/ml in a 50 mM Tris-HCl solution (pH = 7.4) containing 0.2 mg/ml fibronectin. The dispersion state was maintained for 1 minute while this solution was gently stirred by inverting the container. Then, the solution was centrifuged at 2500 rpm for 1 minute (FN immersion operation). After the supernatant was removed, a 50 mM Tris-HCl solution (pH = 7.4) was added so that the cells were dispersed. The dispersion state was maintained for 1 minute while this solution was gently stirred by inverting the container. Then, the solution was centrifuged at 2500 rpm for 1 minute (washing operation). After the supernatant was removed, the cells were dispersed in a 50 mM Tris-HCl solution (pH = 7.4) containing 0.2 mg/ml gelatin. The dispersion state was maintained for 1 minute while this solution was gently stirred by inverting the container. Then, the solution was centrifuged at 2500 rpm for 1 minute (G immersion operation). Subsequently, the washing operation was performed. The FN immersion operation, the washing operation, the G immersion operation, and the washing operation were performed in this order. In this case, a set of the FN immersion operation and the washing operation was defined as one step, and another set of the G immersion operation and the washing operation was defined as one step. Finally, a total of 9 steps, i.e., 5 times of the FN immersion operation and 4 times of the G immersion operation were performed, so that FN-G coated cells were prepared (the thickness of the coating film containing an extracellular matrix component: 8 nm).

### [Evaluation of culture medium consumption / Analysis of gene expression level]

### (Example 1)

First, 7 × 10⁵ HepG2 coated cells were seeded on a membrane filter of Transwell (manufactured by Corning Incorporated; pore size: 0.4 µm, surface area: 0.33 cm²) placed on a 24-well culture plate. Then, 2.5 mL of Eagle's MEM medium containing 10 wt% fetal bovine serum was added, and the coated cells were cultured at 37°C for 1 day. The Transwell in which the coated cells had been seeded was placed on a 6-well culture plate, and 12 mL of Eagle's MEM medium containing 10 wt% fetal bovine serum was added. The coated cells were cultured at 37°C for 8 days without replacing the culture medium, and thus a three-dimensional cell culture construct was produced (the volume of the culture medium during the culture: 170 µL per 1 × 10⁴ cells).

### (Example 2)

A three-dimensional cell culture construct was produced in the same manner as Example 1 except that the coated cells were cultured while the culture medium was replaced every three days (i.e., the period in which the culture medium was not replaced was 2 days) after the Transwell was placed on the 6-well culture plate. In each replacement, almost all of the culture medium included in the well was removed and replaced with a new culture medium (the volume of the culture medium during the culture: 170 µL per 1 × 10⁴ cells).

### (Comparative Example 1)

A three-dimensional cell culture construct was produced in the same manner as Example 1 except that the coated cells were cultured with the Transwell being placed on the 24-well culture plate while the culture medium was replaced after 1 day from the seeding of the coated cells, and then the culture medium was replaced every three days. In each replacement, almost all of the culture medium included in the well was removed and replaced with a new culture medium (the volume of the culture medium during the culture: 36 µL per 1 × 10⁴ cells).

### Evaluation of culture medium consumption

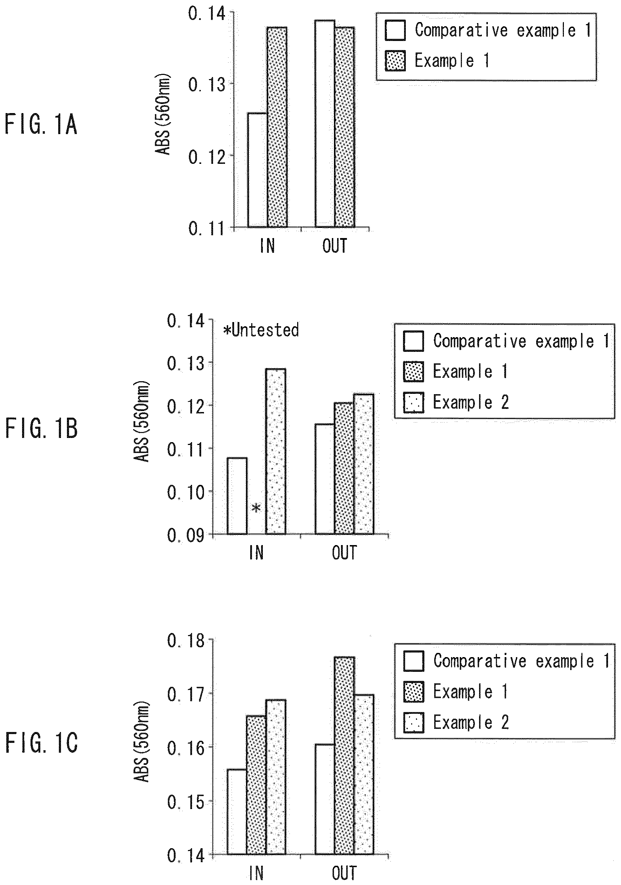

The absorbances (560 nm) of the solution in the inside and the outside of the insert were measured before the replacement of the culture medium. FIG. 1 shows the results. FIGS. 1A to 1C are graphs showing the absorbances of the solution in the inside and the outside of the insert. FIG. 1A shows the absorbances of the solution on the third day of the culture before the replacement of the culture medium. FIG. 1B shows the absorbances of the solution on the seventh day of the culture before the replacement of the culture medium. FIG. 1C shows the absorbances of the solution on the ninth day of the culture. As shown in FIG. 1, on the third day, the seventh day, and the ninth day of the culture, the absorbances of the solution in both the inside and the outside of the insert were lower in Comparative Example 1 (24-well) than in Examples 1 and 2. Thus, it is expected that the culture medium was consumed in Comparative Example 1. Moreover, on the seventh day and the ninth day of the culture, the absorbance of the solution in the outside of the insert in Comparative Example 1 was lower than that of the solution in the inside of the insert in Examples 1 and 2. Thus, it is considered that the whole culture medium was consumed in Comparative Example 1. On the other hand, a difference in absorbance between the inside and the outside of the insert was small in Examples 1 and 2, and particularly almost no difference was found in Example 2.

### Analysis of gene expression level

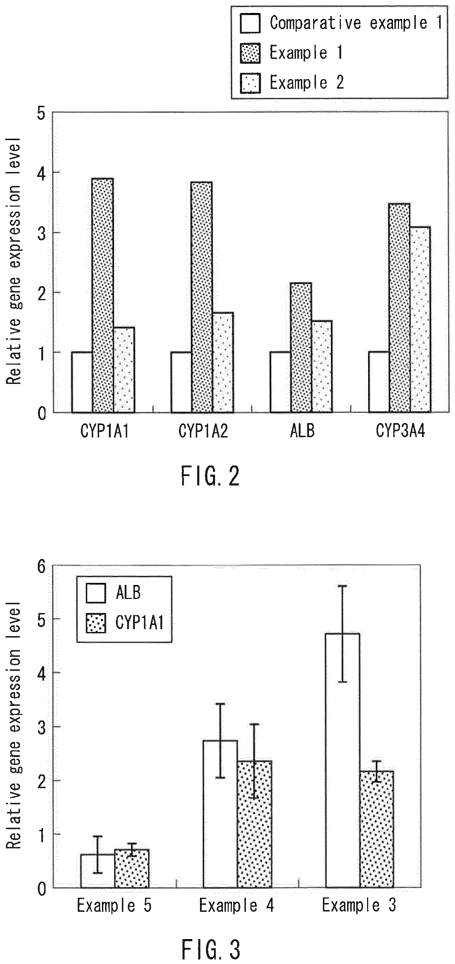

RNA was extracted from each of the samples (three-dimensional cell culture constructs) after the culture for 9 days, and the gene expression levels of CYP1A1, CYP1A2, ALB (albumin), and CYP3A4 were measured by real-time PCR. FIG. 2 shows the results, which are represented by relative values using the gene expression level in Comparative Example 1 as the reference, i.e., 1. As shown in FIG. 2, the CYP activity and the amount of albumin produced of the samples in Examples 1 and 2 were higher than those of the sample in Comparative Example 1. The results indicate that the three-dimensional cell culture constructs produced by the methods in Examples 1 and 2 achieved higher activity.

### [Relationship between volume of culture medium and gene expression level]

In Example 3, the coated cells were cultured under the same conditions as Example 1 except that a 100 mm dish was used instead of the 6-well culture plate (the volume of the culture medium during the culture: 1 mL per 1 × 10⁴ cells). In Example 4, the coated cells were cultured under the same conditions as Example 1. In Example 5, the coated cells were cultured under the same conditions as Example 1 except that a 24-well culture plate was used instead of the 6-well culture plate (the volume of the culture medium during the culture: 36 µL per 1 × 10⁴ cells). The gene expression levels of the three-dimensional cell culture constructs in Examples 3 to 5 were evaluated. FIG. 3 shows the results, which are represented by relative values using the gene expression level measured under the same conditions as Comparative Example 1 (conventional method) as the reference, i.e., 1.

As shown in FIG. 3, the expression level of ALB was higher as the volume of the culture medium became larger. On the other hand, the expression level of CYP1A1 in Example 3 was about the same as that in Example 4. The results indicate the possibility that a three-dimensional cell culture construct with higher activity will be produced by increasing the volume of the culture medium.

### [Relationship between number of cells and gene expression level]

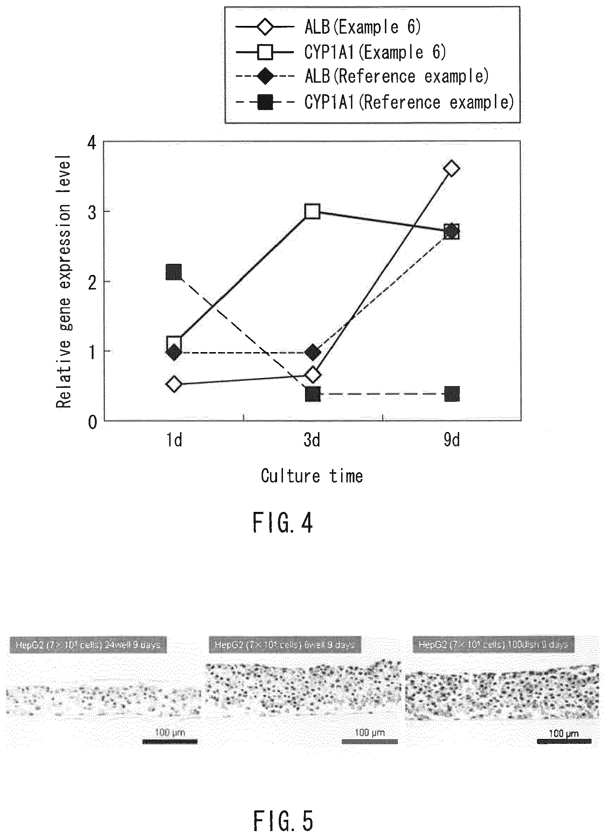

In Example 6, the coated cells were cultured under the same conditions as Example 1. In Reference Example, the coated cells were cultured under the same conditions as Example 1 except that 7 × 10⁵ cells were directly seeded in a well without using an insert. The gene expression levels of the three-dimensional cell culture construct in Example 6 and the cells in Reference Example were evaluated. FIG. 4 shows the results, which are represented by relative values using the gene expression level of a sample after the culture for 9 days under the same conditions as Comparative Example 1 (conventional method) as the reference, i.e., 1.

As shown in FIG. 4, there was no significant difference in the expression level of ALB between Example 6, in which the coated cells were cultured in a layered state, and Reference Example, in which the cells were cultured independently of one another. On the other hand, there was no significant difference in the expression level of CYP1A1 between Example 6 and Reference Example on the first day of the culture. However, in Reference Example, the expression level of CYP1A1 was reduced from the third day of the culture. In Example 6, the expression level of CYP1A1 was raised and kept high even on the ninth day of the culture.

### [Evaluation of three-dimensional cell culture construct]

The three-dimensional cell culture constructs produced in Examples 3 to 5 were evaluated. As shown in FIG. 3, the three-dimensional cell culture constructs of Examples 3 and 4 had higher activity than the three-dimensional cell culture construct of the conventional method. The three-dimensional cell culture construct of Example 5 had substantially the same activity as the three-dimensional cell culture construct of the conventional method. FIG. 5 shows an example of the microphotographs of the three-dimensional cell culture constructs produced in Examples 3 to 5. As shown in FIG. 5, both the three-dimensional cell culture construct of Example 4 using the 6-well culture plate and the three-dimensional cell culture construct of Example 3 using the 100 mm dish had a large thickness and a large number of layers compared to the three-dimensional cell culture construct of Example 5 using the 24-well culture plate.

### [Relationship between period of continuous use of culture medium and gene expression level]

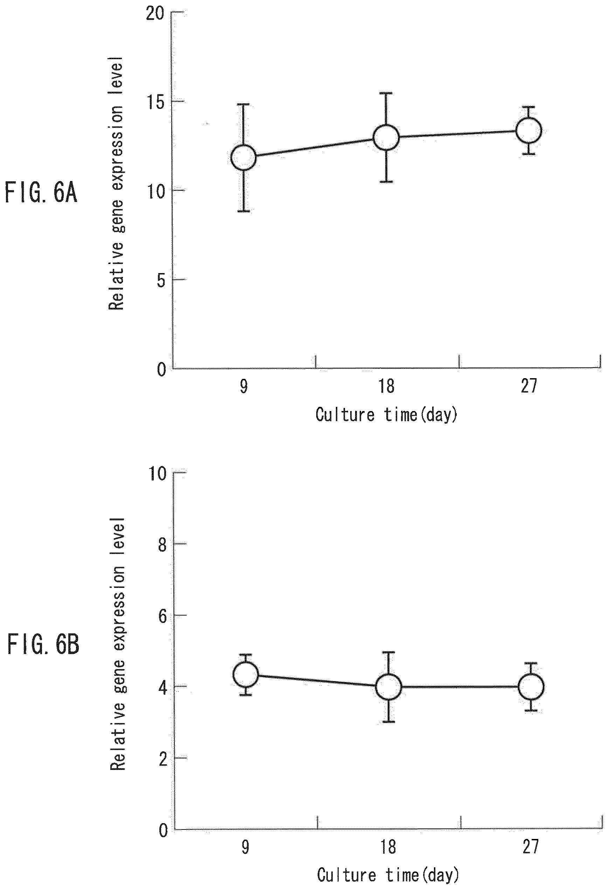

In Example 7, the coated cells were cultured for 27 days under the same conditions (the volume of the culture medium during the culture: 1 mL per 1 × 10⁴ cells, the culture medium was not replaced from the second day of the culture) as Example 3. With respect to the samples on the 9th day, the 18th day, and the 27th day of the culture, the gene expression levels of CYP1A1 and ALB were measured by real-time PCR. FIG. 6 shows the results, which are represented by relative values using the gene expression level of a sample after the culture for 9 days by the conventional method (the volume of the culture medium during the culture: 36 µL per 1 × 10⁴ cells, the culture medium was replaced every three days from the second day) as the reference, i.e., 1. FIG. 6A is a graph showing the expression level of ALB. FIG. 6B is a graph showing the expression level of CYP1A1. As shown in FIG. 6, both ALB and CYP1A1 maintained a high expression level in a culture period of 27 days. The results confirm that even if the coated cells are cultured by using the culture medium continuously for 27 days without replacement, the three-dimensional cell culture construct can maintain high activity.

## Claims

1. A method for producing a three-dimensional cell culture construct comprising at least two or more laminated cell layers, the method comprising:
seeding coated cells in which a cell surface is coated with a coating film containing an extracellular matrix component; and
culturing the seeded coated cells in a culture medium,
wherein the culture process of the coated cells includes culturing the coated cells in 170 µL. or more of the culture medium per 1 × 10⁴ cells.

2. The method according to claim 1, wherein the coating film containing an extracellular matrix component comprises a film containing a material A and a film containing a material B that interacts with the material A, and, the combination of the material A and the material B is a combination of a protein or polymer having an RGD sequence and a protein or polymer that interacts with the protein or polymer having an RGD sequence.

3. The method according to claim 1 or claim 2, wherein the thickness of the coating film is 1 nm to 1 × 10³ nm.

4. The method according to any one of claims 1 to 3, wherein the coating film is 5 layers or more.

## Patentansprüche

1. Verfahren zum Herstellen eines dreidimensionalen Zellkulturkonstrukts, umfassend mindestens zwei oder mehr laminierte Zellschichten, wobei das Verfahren Folgendes umfasst:
Impfen von beschichteten Zellen, wobei eine Zelloberfläche mit einem eine extrazelluläre Matrixkomponente enthaltenden Beschichtungsfilm beschichtet wird; und
Kultivieren der geimpften beschichteten Zellen in einem Kulturmedium,
wobei der Kulturprozess der beschichteten Zellen das Kultivieren der beschichteten Zellen in 170 µl oder mehr des Kulturmediums pro 1 x 10⁴ Zellen einschließt.

2. Verfahren nach Anspruch 1, wobei der eine extrazelluläre Matrixkomponente enthaltende Beschichtungsfilm einen Film, der ein Material A enthält, und einen Film, der ein Material B enthält, das mit dem Material A interagiert, umfasst und die Kombination des Materials A und des Materials B eine Kombination eines Proteins oder Polymers mit einer RGD-Sequenz und eines Protein oder Polymers, das mit dem Protein oder Polymer mit einer RGD-Sequenz interagiert, ist.

3. Verfahren nach Anspruch 1 oder Anspruch 2, wobei die Dicke des Beschichtungsfilms 1 nm bis 1 x 10³ nm beträgt.

4. Verfahren nach einem der Ansprüche 1 bis 3, wobei der Beschichtungsfilm aus 5 Schichten oder mehr besteht.

## Revendications

1. Procédé de fabrication de corps de culture cellulaire tridimensionnelle comprenant au moins deux ou plus de deux couches de cellules stratifiées, le procédé comprenant :
l'ensemencement de cellules revêtues dans lesquelles une surface cellulaire est revêtue avec un film de revêtement contenant un composant de matrice extracellulaire ; et
la mise en culture des cellules revêtues ensemencées dans un milieu de culture,
dans lequel le procédé de culture des cellules revêtues inclut la mise en culture des cellules revêtues dans 170 µl ou plus du milieu de culture pour 1 x 10⁴ cellules.

2. Procédé selon la revendication 1, dans lequel le film de revêtement contenant un composant de matrice extracellulaire comprend un film contenant un matériau A et un film contenant un matériau B qui interagit avec le matériau A, et, la combinaison du matériau A et du matériau B est une combinaison d'une protéine ou d'un polymère ayant une séquence RGD et d'une protéine ou d'un polymère qui interagit avec la protéine ou le polymère ayant une séquence RGD.

3. Procédé selon la revendication 1 ou la revendication 2, dans lequel l'épaisseur du film de revêtement est de 1 nm à 1 x 10³ nm.

4. Procédé selon l'une quelconque des revendications 1 à 3, dans lequel le film de revêtement est 5 couches ou plus.
